# EUROPEAN PATENT APPLICATION

(11) **EP 2 335 686 A1**
(43) Date of publication of application: **22.06.2011**
(21) Application number: 09180127.4
(22) Date of filing: 21.12.2009
(51) Int. Cl.: A61K 9/107, A61K 9/00, A61K 47/10, A61K 47/26, A61K 31/343

(54) **An aqueous intravenous nanosuspension with reduced adverse effects**

(71) Applicant: LEK Pharmaceuticals d.d., 1526 Ljubljana (SI)
(72) Inventor: The designation of the inventor has not yet been filed
(74) Representative: Kosak, Alenka

(57) **Abstract**

The present invention relates to an aqueous nanosuspension formulation comprising a drug possessing low intrinsic water solubility, e.g. amiodarone. It also provides a method for the preparation of intravenous formulation comprising amiodarone, with markedly decreased adverse effects.

## Description

### Background of the Invention

The present invention relates to aqueous nanosuspensions of poorly water soluble drugs suitable for intravenous administration, to methods of preparing said nanosuspensions, and to the use of said nanosuspensions. In particular, the present invention relates to aqueous nanosuspensions comprising amiodarone.

Amiodarone has been used as an anti-arrhythmic drug since the 1970s and has had an established role in the treatment of ventricular tachyarrhythmias. Although considered to be a class III anti-arrhythmic, amiodarone also has class I, II and IV actions, which provides a unique pharmacological and anti-arrhythmic profile. In contrast to oral amiodarone, which takes days to work in ventricular tachyarrhythmias, intravenously administered amiodarone has immediate effect and can be used in life threatening ventricular arrhythmias. Intravenous amiodarone administration can help in many cases, for example if administered after out-of-hospital cardiac arrest due to ventricular fibrillation, it improves survival to hospital admission. Moreover, it reduces chances of occurrence of sudden death after myocardial infarction, a benefit, which is predominantly observed in patients with preserved cardiac function. Meta-analysis of different amiodarone trials established a survival benefit of amiodarone in heart failure. Amiodarone is effective in chronic and recent onset atrial fibrillation and orally or i.v. for atrial fibrillation after heart surgery. In atrial fibrillation amiodarone is more effective than or equi-effective with flecainide, quinidine, racemic sotalol, propafenone and diltiazem, and is therefore being considered as a first line therapy. Amiodarone is also safe and effective in controlling refractory tachyarrhythmias in infants and is safe after cardiac surgery.

Amiodarone free base has an extremely low estimated intrinsic solubility in water. The free base is neutral and by protonation with acid it is converted to a more water-soluble trialkylammonium ion. However, the hydrochloride salt (used in the current commercial product, Cordarone®) is not appreciably soluble in water at moderately low pH (3.8-4.5), therefore the formulation also contains polysorbate 80 as a surfactant for better dissolution and solubilization of the drug, as described in EP 835105 B1.

In addition to commercially available product, several other formulations of amiodarone for parenteral administration have been publicly introduced. US patent 5234949 discloses a parenteral solution comprising 25 to 50 mg/mL amiodarone in 0.05 to 0.1 M acetate buffer having a pH of about 3.5 to 3.8. US patent 6143778 describes a composition for parenteral administration comprising from 1.5 to 8% by weight of an active principle consisting of amiodarone or one of the pharmaceutically acceptable salts thereof which remains stable upon dilution down to a concentration of from 0.05 to 0.2% in active principle, wherein the pharmaceutical composition further comprises a physiologically acceptable buffer solution capable of solubilizing the active principle and of maintaining the pH of the composition between 2.4 and 3.8; and from 0.5 to 2% by weight of a nonionic hydrophillic surfactant. Furthermore, a parenteral solution for intravenous administration without dilution comprising as an active ingredient, amiodarone in a concentration range from 0.2 to 10 mg/ml and a buffer solution selected from the group consisting of lactate buffer, methanesulfonate buffer, and combinations thereof, the solution being free of surfactant and having a pH within the range of from approximately 2.5 to approximately 4.5 has been the subject matter of the US patent 6479541. Moreover, US patent 7067143 discloses a solution for intravenous administration comprising amiodarone, as an active ingredient, solubilized to a concentration range of from 0.2 to 6 mg/ml, in a solution of water for injection and a nonionic surfactant; optionally, an osmotic agent; and wherein the solution requires no dilution before administering and has a pH within the range of from about 2.9 to about 3.2. Finally, aqueous parenteral formulation containing amiodarone and a sulfoalkyl ether cyclodextrin, which does not require a surfactant and does not precipitate upon dilution with distilled water or other pharmaceutically acceptable liquid carrier, is disclosed in the US patent 6869939.

The object of the present invention is to provide an improved formulation of a drug possessing low water solubility, e.g. amiodarone, which would be stable and could be administered at a physiologically acceptable or relevant pH, and would not have to be diluted prior to administration. Moreover, it would be desirable to provide such a formulation comprising a drug possessing low water solubility, e.g. amiodarone, that would reduce or eliminate the potential adverse effects induced by excipients and/or by the precipitation of the drug upon administration.

### Summary of Invention

The composition of the present invention is typically in the form of an aqueous nanosuspension, which allows that only a small amount of stabilizing agent is required to successfully prevent particle agglomeration.

Said composition is described by the following embodiments of the invention. In particular, various aspects, advantageous features and preferred embodiments of the present invention will be summarized in the following items which, respectively alone or in combination, further contribute to solving the object of the invention:
1. An aqueous nanosuspension for use in intravenous administration comprising an active ingredient having intrinsic solubility in water of less than 1 mg/mL, and a stabilizing agent, wherein the stabilizing agent is present in an amount of less than 5 % w/w, in a therapeutic use of said active ingredient.
2. An aqueous nanosuspension for use in intravenous administration comprising an active ingredient having intrinsic solubility in water of less than 0.5 mg/mL, and a stabilizing agent, wherein the stabilizing agent is present in an amount of less than 5 % w/w, in a therapeutic use of said active ingredient.
3. An aqueous nanosuspension for use in intravenous administration comprising an active ingredient having intrinsic solubility in water of less than 0.1 mg/mL, and a stabilizing agent, wherein the stabilizing agent is present in an amount of less than 5 % w/w, in a therapeutic use of said active ingredient.
4. The aqueous nanosuspension according to any of items 1 to 3, wherein the stabilizing agent is present in an amount of less than 2 % w/w.
5. The aqueous nanosuspension according to any of preceding items, wherein the active ingredient is selected from a group consisting of amiodarone, diazepam, phenytoin, vancomycin and clarithromycin, preferably the active ingredient is selected from a group consisting of amiodarone, diazepam and clarithromycin.
6. The aqueous nanosuspension according to any of preceding items, wherein the stabilizing agent is selected from the group consisting of polysorbate 80, polysorbate 20, hydroxylpropylmethyl cellulose, poloxamer 188, polyvinylpirolydone, Labrasol®, Gelucire®, gelatin, lecithin (phosphatides), gum acacia, cholesterol, tragacanth, polyoxyethylene alkyl ethers, polyoxyethylene castor oil derivatives, polyoxyethylene sorbitan fatty acid esters, sorbitan fatty acid esters, polyethylene glycols, polyoxyethylene stearates, mono and diglycerides, colloidal silicon dioxide, sodium dodecylsulfate, magnesium aluminum silicate, triethanolamine, stearic acid, calcium stearate, glycerol monostearate, cetostearyl alcohol, cetomacrogol emulsifying wax, short and medium chain alcohols, Labrafil®, Purol-oleique® and polyvinyl alcohol, preferably polysorbate 80, polyvinyl alcohol and poloxamer 188, in particular polysorbate 80.
7. The aqueous nanosuspension according to any of preceding items, further comprising one or more pharmaceutically acceptable excipients.
8. The aqueous nanosuspension according to any of preceding items, wherein the particles have an average size, measured by photon correlation spectroscopy, in the range of from 10 nm to 1000 nm.
9. The aqueous nanosuspension according to any preceding items, wherein the particles have an average size, measured by photon correlation spectroscopy, in the range of from 10 nm to 500 nm.
10. The aqueous nanosuspension according to items 1 to 9, wherein said aqueous nanosuspension is stable in a pH range from 2 to 10, preferably in a pH range from 5 to 8.
11. An aqueous nanosuspension for use in intravenous administration comprising amiodarone and a stabilizing agent, wherein the stabilizing agent is present in an amount less than 5 % w/w, in a therapeutic use of amiodarone.
12. The aqueous nanosuspension according to item 11, wherein the stabilizing agent is present in an amount of less than 2 % w/w.
13. The aqueous nanosuspension according to items 11 and 12, wherein the stabilizing agent is selected from the group consisting of polysorbate 80, polyvinyl alcohol and poloxamer 188, preferably the stabilizing agent is polysorbate 80.
14. The aqueous nanosuspension according to items 11 to 13, further comprising one or more pharmaceutically acceptable excipients.
15. The aqueous nanosuspension according to item 14, wherein the pharmaceutically acceptable excipients are selected from the group consisting of protective agents and viscosity increasing agents.
16. The aqueous nanosuspension according to item 15, wherein the protective agent is benzyl alcohol, and the viscosity increasing agent is glycerol.
17. The aqueous nanosuspension according to any of items 11 to 16, wherein the particles have an average size, measured by photon correlation spectroscopy, in the range of from 100 nm to 1000 nm.
18. The aqueous nanosuspension according to any of items 11 to 17, wherein the particles have an average size, measured by photon correlation spectroscopy, in the range of from 200 nm to 500 nm.
19. The aqueous nanosuspension according to any of items 11 to 18, wherein said aqueous nanosuspension is stable in a pH range from 2 to 10, preferably in a pH range from 5 to 8.
20. A process for preparing an aqueous nanosuspension for use in intravenous administration, the process comprising:
   (i) providing an active ingredient having intrinsic solubility in water of less than 1 mg/mL,
   (ii) providing aqueous solution of stabilizing agent and optionally one or more pharmaceutically acceptable excipients,
   (iii) suspending the active ingredient in the obtained aqueous solution of stabilizing agent and optionally one or more pharmaceutically acceptable excipients, and
   (iv) subjecting the suspension obtained at step (iii) to high pressure and/or physical force to provide said aqueous nanosuspension for a therapeutic use of said active ingredient.
21. The process according to item 20, wherein the active ingredient is selected from a group consisting of amiodarone, diazepam, phenytoin, vancomycin and clarithromycin, preferably the active ingredient is selected from a group consisting of amiodarone, diazepam and clarithromycin.
22. The process according to item 21, wherein the stabilizing agent is selected from the group consisting of polysorbate 80, polysorbate 20, hydroxylpropylmethyl cellulose, poloxamer 188, polyvinylpirolydone, Labrasol®, Gelucire®, gelatin, lecithin (phosphatides), gum acacia, cholesterol, tragacanth, polyoxyethylene alkyl ethers, polyoxyethylene castor oil derivatives, polyoxyethylene sorbitan fatty acid esters, sorbitan fatty acid esters, polyethylene glycols, polyoxyethylene stearates, mono and diglycerides, colloidal silicon dioxide, sodium dodecylsulfate, magnesium aluminum silicate, triethanolamine, stearic acid, calcium stearate, glycerol monostearate, cetostearyl alcohol, cetomacrogol emulsifying wax, short and medium chain alcohols, Labrafil®, Purol-oleique® and polyvinyl alcohol, preferably polysorbate 80, polyvinyl alcohol and poloxamer 188.
23. The process according to items 20 to 22, wherein the particles of the provided aqueous nanosuspension have an average size of from 10 nm to 1000 nm.
24. The process according to item 20 to 23, wherein the particles of the provided aqueous nanosuspension have an average size of from 10 nm to 500 nm.
25. A process for preparing an aqueous nanosuspension for use in intravenous administration, the process comprising:
   (i) providing amiodarone,
   (ii) providing aqueous solution of stabilizing agent selected from the group consisting of polysorbate 80, polyvinyl alcohol and poloxamer 188, and optionally one or more pharmaceutically acceptable excipients,
   (iii) suspending amiodarone in the aqueous solution obtained at step (ii), and
   (iv) subjecting the suspension obtained at step (iii) to high pressure and/or physical force to provide said aqueous nanosuspension for a therapeutic use of amiodarone.
26. The process according to item 25, wherein the pharmaceutically acceptable excipients are selected from the group consisting of protective agents and viscosity increasing agents.
27. The process according to item 26, wherein the protective agent is benzyl alcohol, and the viscosity increasing agent is glycerol.
28. The process according to items 25 to 27, wherein the particles of the provided aqueous nanosuspension have an average size of from 10 nm to 1000 nm.
29. The process according to item 25 to 28, wherein the particles of the provided aqueous nanosuspension have an average size of from 10 nm to 500 nm.
30. A pharmaceutical composition comprising the aqueous nanosuspension according to any of items 1 to 19.
31. Use of aqueous nanosuspension comprising an active ingredient having intrinsic solubility in water of less than 1 mg/mL, and a stabilizing agent, wherein the stabilizing agent is present in an amount of less than 5 % w/w, for making a pharmaceutical formulation for intravenous administration for a therapy or prophylaxis with said active ingredient.
32. Use of aqueous nanosuspension comprising an active ingredient having intrinsic solubility in water of less than 0.5 mg/mL, and a stabilizing agent, wherein the stabilizing agent is present in an amount of less than 5 % w/w, for making a pharmaceutical formulation for intravenous administration for a therapy or prophylaxis with said active ingredient.
33. Use of aqueous nanosuspension comprising an active ingredient having intrinsic solubility in water of less than 0.1 mg/mL, and a stabilizing agent, wherein the stabilizing agent is present in an amount of less than 5 % w/w, for making a pharmaceutical formulation for intravenous administration for a therapy or prophylaxis with said active ingredient.
34. Use according to items 31 to 33, wherein the stabilizing agent is present in an amount of less than 2 % w/w.
35. Use according to items 31 to 34, wherein the active ingredient is selected from a group consisting of amiodarone, diazepam, phenytoin, vancomycin and clarithromycin, preferably the active ingredient is selected from a group consisting of amiodarone, diazepam and clarithromycin.
36. Use according to items 31 to 35, wherein the stabilizing agent is selected from the group consisting of polysorbate 80, polysorbate 20, hydroxylpropylmethyl cellulose, poloxamer 188, polyvinylpirolydone, Labrasol®, Gelucire®, gelatin, lecithin (phosphatides), gum acacia, cholesterol, tragacanth, polyoxyethylene alkyl ethers, polyoxyethylene castor oil derivatives, polyoxyethylene sorbitan fatty acid esters, sorbitan fatty acid esters, polyethylene glycols, polyoxyethylene stearates, mono and diglycerides, colloidal silicon dioxide, sodium dodecylsulfate, magnesium aluminum silicate, triethanolamine, stearic acid, calcium stearate, glycerol monostearate, cetostearyl alcohol, cetomacrogol emulsifying wax, short and medium chain alcohols, Labrafil®, Purol-oleique® and polyvinyl alcohol, preferably polysorbate 80, polyvinyl alcohol and poloxamer 188, in particular polysorbate 80.
37. Use according to items 31 to 36, wherein said aqueous nanosuspension further comprises one or more pharmaceutically acceptable excipients.
38. Use according to items 31 to 37, wherein the particles of said aqueous nanosuspension have an average size, measured by photon correlation spectroscopy, in the range of from 10 nm to 1000 nm.
39. Use according to items 31 to 38, wherein the particles of said aqueous nanosuspension have an average size, measured by photon correlation spectroscopy, in the range of from 10 nm to 500 nm.
40. Use according to items 31 to 39, wherein said aqueous nanosuspension is stable in a pH range from 2 to 10, preferably in a pH range from 5 to 8.
41. Use of aqueous nanosuspension comprising amiodarone, and a stabilizing agent, wherein the stabilizing agent is present in an amount of less than 5 % w/w, for making a pharmaceutical formulation for intravenous administration for a therapy or prophylaxis with amiodarone.
42. Use according to item 41, wherein the stabilizing agent is present in an amount of less than 2 % w/w.
43. Use according to items 41 or 42, wherein the stabilizing agent is selected from the group consisting of polysorbate 80, polyvinyl alcohol and poloxamer 188, preferably the stabilizing agent is polysorbate 80.
44. Use according to items 41 to 43, wherein said aqueous nanosuspension further comprises one or more pharmaceutically acceptable excipients.
45. Use according to item 44, wherein the pharmaceutically acceptable excipients are selected from the group consisting of protective agents and viscosity increasing agents.
46. Use according to item 45, wherein the protective agent is benzyl alcohol, and the viscosity increasing agent is glycerol.
47. Use according to items 41 to 46, wherein the particles of said aqueous nanosuspension have an average size, measured by photon correlation spectroscopy, in the range of from 10 nm to 1000 nm.
48. Use according to items 41 to 47, wherein the particles of said aqueous nanosuspension have an average size, measured by photon correlation spectroscopy, in the range of from 10 nm to 500 nm.
49. Use according to items 41 to 48, wherein said aqueous nanosuspension is stable in a pH range from 2 to 10, preferably in a pH range from 5 to 8.

In contrast to the marketed product Cordarone^{®}, the aqueous nanosuspension of the present invention comprises substantially lower amounts of Polysorbate 80. This is advantageous since glycol ethers and their derivatives such as polyethylene glycols (PEGs) or polysorbates (Tweens) are known to contribute to pain on injection, and may also induce anaphylactic reactions. In addition, Polysorbate 80 has also been associated with cardiac depressions, causing hypotension (Gough et al., "Hypotensive Action of Commercial Intravenous Amiodarone and Polysorbate 80 in Dogs," Journal of Cardiovascular Pharmacology, 1982, 375-380). An additional advantage over the commercialized product is that the aqueous nanosuspension of the present invention does not have to be diluted prior to administration, which is at most important when quick administration of amiodarone is required in life threatening situations.

Furthermore, the aqueous nanosuspension of the present invention is less pH-dependent. As a consequence, possible stability issues, e.g. precipitation and possible agglomeration of the drug, that may arise due to a change in pH upon dilution of pH-dependent systems with body fluids, can be avoided.

Moreover, since in the aqueous nanosuspension formulation of the present invention amiodarone is in a stable solid/crystalline form its precipitation following dilution with the blood does not occur. As a consequence side effects that result due to precipitation, for example vascular complications, lack of therapeutic activity on precipitation after injection, pain and inconvenience for the patients, are prevented.

In contrast to cyclodextrin formulation, the release of a drug from the aqueous nanosuspension is faster, since it is governed by dissolution and not by dissociation equilibrium as is the case for cyclodextrin complexes. Consequently, the well-defined amount of the released drug provides a more convenient dosage regimen. In addition, compared to formulations comprising cyclodextrins the aqueous nanosuspensions of the present invention are also cheaper and easier to produce and easier to handle.

### Brief Description of the Figures

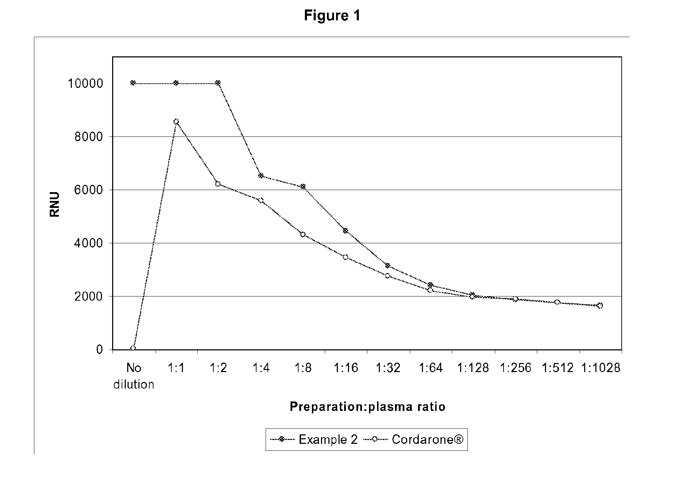

FIGURE 1: Turbidity of amiodarone formulation diluted with human plasma measured using Nephelostar Galaxy (BMG Labtech) with laser intensity of 90%, beam focus 1.5mm, measured time per well 0.1 second. Results are shown as mean values of 2-3 independent measurements (RNU = relative nephelometric units).

### Detailed Description of the Invention

Drugs possessing low intrinsic water solubility are difficult to formulate in a water-based parenteral formulation that is sufficiently concentrated and stable in a medium having a physiologically acceptable pH. Certain intravenous formulations of poorly water-soluble drugs exhibit adverse effects which are a consequence of high concentrations of excipients (e.g. solubilizers, surfactants) used for the solubilization of the drugs, or a consequence of drug precipitation upon administration. When a solution of the drug in a surfactant-based vehicle is diluted in the bloodstream the pH of the formulation approaches that of the blood, which might cause precipitation of the drug, especially if said drug possesses a pH-dependent solubility profile. In addition, precipitation can also occur due to the fact that upon administration both concentration of the drug and the one of surfactant decrease, which may result in a physically unstable solution.

The inventors of the present invention extensively studied a possibility to make an improved formulation of poorly water soluble drugs, such as amiodarone, which would overcome pH and dilution-dependent stability issues, and issues arising due to usage of higher concentrations of solubilizers. As a result, it was found that this can be achieved by providing an aqueous nanosuspension and a formulation comprising the same, the aqueous nanosuspension comprising the poorly water soluble drug and one or more pharmaceutically acceptable excipients, while carefully observing size characteristics of drug particles. In the aqueous nanosuspension of the present invention the drug is in a stable solid/crystalline form, therefore the additional precipitation of the drug following dilution with the blood (body fluids) is prevented.

Nanosuspensions are dispersed systems of solid-in-liquid or solid-in-semisolid, the dispersed phase comprising drug particles ranging in size approximately from nanometer to micron. Usually an excipient, e.i. stabilizing agent, is required to prevent particle agglomeration and thusly provide a stable nanosuspension.

The most important component of the aqueous nanosuspension of the present invention is the presence of a drug possessing low intrinsic water solubility, that is a drug having the intrinsic water solubility of less than 1 mg/mL. Examples of such drugs are amiodarone (aqueous solubility = 18.6 ng/mL), diazepam (aqueous solubility at room temperature = 57 ng/mL), propofol (aqueous solubility at room temperature = 16.4 ng/mL), phenytoin, cytostatics (e.g. anthracyclines) and antibiotics (e.g. clarithromycin, vancomycin), and others. A preferred drug possessing low intrinsic water solubility is amiodarone. Pharmaceutically acceptable salts of the foregoing, said salts having solubility in water of less than 1 mg/mL are also contemplated for addition to the aqueous nanosuspensions as described herein.

In addition to the drug possessing low intrinsic water solubility, the aqueous nanosuspension of the present invention contains a pharmaceutically acceptable stabilizing agent. A non-limiting specific examples of suitable nanosuspension stabilizing excipients are for example polysorbate 80, polysorbate 20, hydroxylpropylmethyl cellulose, poloxamer 188, polyvinylpirolydone, Labrasol®, Gelucire®, gelatin, lecithin (phosphatides), gum acacia, cholesterol, tragacanth, polyoxyethylene alkyl ethers, polyoxyethylene castor oil derivatives, polyoxyethylene sorbitan fatty acid esters, sorbitan fatty acid esters, polyethylene glycols, polyoxyethylene stearates, mono and diglycerides, colloidal silicon dioxide, sodium dodecylsulfate, magnesium aluminum silicate, triethanolamine, stearic acid, calcium stearate, glycerol monostearate, cetostearyl alcohol, cetomacrogol emulsifying wax, short and medium chain alcohols, Labrafil®, Purol-oleique® and polyvinyl alcohol. The concentration of the stabilizing agent is usually about 1% (w/w) of the aqueous nanosuspension, which is considerably lower than the amount required for the solubilization of the drug. The preferred stabilizing agent is polysorbate 80. The concentration of the stabilizing agent depends on its nature, in general the concentration is below 10% (w/w), more preferably below 3% (w/w), in particular about 1 % (w/w) of the aqueous nanosuspension.

The term "about" as used herein means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%.

The term "stable aqueous nanosuspension" as used herein means that drug particles comprising said aqueous nanosuspension do not form larger particles, which would exceed the average particle size for more than 2 or 3 times. Drug particles may form loose aggregates which are deagglomerated by shaking to yield particles in the desired particle size interval. Drug particles may sediment, but are redispersed by shaking to yield particles in the desired particle size interval.

The aqueous nanosuspension of the present invention may additionally comprise one or more additional pharmaceutically acceptable excipients, for example a preserving or a protective agent such as a bactericide, e.g. benzyl alcohol, a viscosity increasing agent such as glycerol, osmolarity regulating agent, e.g. sodium chloride, buffers, and crystallization inhibitors.

In another embodiment the particle size of a drug formulated in the aqueous nanosuspension of the present invention is in the range from 10 to 4000 nanometers, preferably in the range from 10 to 1000 nanometers, more preferably in the range from 10 to 500 nanometers, measured for example by photon correlation spectroscopy.

The aqueous nanosuspension of the present invention may be formulated as a bolus injection or intravenous infusion. Furthermore it can be formulated as powder for reconstitution, which upon reconstitution with water or other suitable media yields a bolus injection or intravenous infusion.

In yet another aspect the present invention relates to a process of preparing aqueous nanosuspensions comprising a drug possessing low intrinsic water solubility. In order to prepare the aqueous nanosuspension comprising a drug possessing low intrinsic water solubility, for example amiodarone, first an aqueous solution of a suitable surfactant, for example polysorbate 80 is prepared, to which a suitable amount of the drug, e.g. amiodarone, diazepam or clarithromycin, is added to form a suspension. In the final step, physical force, e.g. ball milling, rotor-stator or high pressure homogenization are used to prepare aqueous nanosuspensions with drug particles having the desired particle sizes. The process parameters of each method are optimized to yield the desired particle size. Other methods for the preparation of nanosuspension, e.g. precipitation from solution, emulsion or microemulsion with antisolvents, reaction conditions or by using various techniques such as spray-drying, freeze-drying, spray-freeze-drying, electro-spraying, supercritical fluid- and microfluidic technology, or combination of the methods can also be used to prepare aqueous nanosuspension with desired particle size.

In a preferred embodiment of the present invention, aqueous nanosuspension of amiodarone is prepared by wet ball milling the amiodarone suspension obtained by adding amiodarone to an aqueous solution of polysorbate 80. Particle size of milled amiodarone can be varied according to the milling parameters used, suitable particle sizes obtained are around 300 to 400 nm, measured by photon correlation spectroscopy.

In another preferred embodiment of the present invention, aqueous nanosuspension of amiodarone is prepared by milling the amiodarone suspension obtained by adding amiodarone to an aqueous solution of polysorbate 80 in a high pressure homogenizer. Milling the suspension in a high pressure homogenizer can be an iterative process starting, for example with 2 cycles at 300 bar, followed by 2 cycles at 1000 bar, which are further followed by 20 cycles at 1500 bar. In this way suitable particle size of amiodarone, e.g. around 300 to 400 nm, can be obtained, measured by photon correlation spectroscopy.

In another preferred embodiment of the present invention, aqueous nanosuspension of amiodarone is prepared by wet ball milling the amiodarone suspension obtained by adding amiodarone to an aqueous solution of polysorbate 80. The aqueous nanosuspension obtained is then further subjected to high pressure homogenization to gain a aqueous nanosuspension with smaller average size of amiodarone particles, for example around 250 nm.

In yet another preferred embodiment of the present invention, aqueous nanosuspension of diazepam is prepared by wet ball milling the diazepam suspension obtained by adding diazepam to an aqueous solution of polysorbate 80. Particle size of milled diazepam can be varied according to the milling parameters used, suitable particle sizes obtained are around 300 to 400 nm, measured by photon correlation spectroscopy.

In another preferred embodiment of the present invention, aqueous nanosuspension of diazepam is prepared by milling the diazepam suspension obtained by adding diazepam to an aqueous solution of polysorbate 80 in a high pressure homogenizer. Milling the suspension in a high pressure homogenizer can be an iterative process starting, for example with 2 cycles at 300 bar, followed by 2 cycles at 1000 bar, which are further followed by 20 cycles at 1500 bar. In this way suitable particle size of diazepam, e.g. around 300 to 400 nm, can be obtained, measured by photon correlation spectroscopy.

In another preferred embodiment of the present invention, aqueous nanosuspension of diazepam is prepared by wet ball milling the diazepam suspension obtained by adding diazepam to an aqueous solution of poloxamer 188. The aqueous nanosuspension obtained is then further subjected to high pressure homogenization to gain a aqueous nanosuspension with smaller average size of diazepam particles, for example around 300 nm.

In another preferred embodiment of the present invention, aqueous nanosuspension of clarithromycin is prepared by wet ball milling the clarithromycin suspension obtained by adding clarithromycin to an aqueous solution of polyvinyl alcohol. The aqueous nanosuspension obtained is then further subjected to high pressure homogenization to gain a aqueous nanosuspension with smaller average size of clarithromycin particles, for example around 350 nm.

In another aspect the present invention is related to the use of aqueous nanosuspension comprising amiodarone for the treatment of arrhythmias, for example ventricular tachyarrhythmias. Amiodarone shows considerable interindividual variation in response. Thus, although a starting dose adequate to suppress life-threatening arrhythmias is needed, close monitoring with adjustment of dose as needed is essential. When administering amiodarone in a form of intravenous infusion, the recommended dose is about 1000 mg over the first 24 hours of therapy. In case of a bolus injection 150 mg of amiodarone is usually administered when treating arrhythmias, while for reanimation purposes a 300 mg dose is administered.

In yet another aspect the present invention is related to the use of aqueous nanosuspension comprising diazepam for the treatment of muscle spasms, alcohol dependency, anxiety and epilepsy.

In yet another aspect the present invention is related to the use of aqueous nanosuspension comprising clarithromycin for the treatment of bacterial infections, for example respiratory tract infections, dermatological infections, Helicobacter pylori infections and otitis.

### Examples:

Aqueous nanosuspensions of amiodarone were prepared by subjecting the amiodarone suspension obtained by adding amiodarone to an aqueous solution of polysorbate 80 to wet ball milling and/or to high pressure homogenization. An average size of milled amiodarone particles measured by photon correlation spectroscopy varied between 200 and 400 nm according to the milling parameters used.

The turbidity of aqueous nanosuspension was compared with the turbidity of the commercial preparation (Cordarone^{®}, see Example 5) to get an insight into precipitation process that might occur when formulations are diluted when applied to the bloodstream.

In addition, adverse effects of aqueous nanosuspension against Cordarone^{®} were assessed by injecting both compositions to mice and observing changes.

### Example 1:

In a glass beaker 2.5 g of polysorbate 80 were mixed with 35 g of water for injections to prepare a clear, yellowish solution. 12.5 g of amiodarone were suspended in the prepared polysorbate solution and milled in a high pressure homogenizer at 300 bar for 2 cycles, 1000 bar for 2 cycles and 1500 bar for 20 cycles. The prepared aqueous nanosuspension had an average particle size of 400 nanometers as measured with a photon correlation spectroscopy (PCS) method.

### Example 2:

In a glass beaker 2 g of polysorbate 80 were mixed with 10 g of water for injections to prepare a clear, yellowish solution. 10 g of amiodarone were suspended in the prepared polysorbate solution and milled in a wet ball mill to prepare an aqueous nanosuspension with average particle size of around 300 nanometers measured with PCS method. The resulting suspension was further diluted with water for injections to prepare an aqueous nanosuspension with amiodarone concentration of 50 mg/mL. The average size of amiodarone particle did not change after the dilution, as verified with PCS method.

### Example 3:

An aqueous nanosuspension of amiodarone was prepared as described in the example 2. The resulting aqueous nanosuspension was further homogenized by means of high pressure homogenizer at 1500 bar for 10 cycles to obtain an aqueous nanosuspension of amiodarone with average particle size of 250 nanometers as measured with a PCs method.

### Example 4:

In a glass beaker 2 g of polysorbate 80 were mixed with 10 g of water for injections to prepare a clear, yellowish solution. 10 g of amiodarone were suspended in the prepared polysorbate solution and milled in a wet ball mill to prepare an aqueous nanosuspension with average particle size of around 300 nanometers measured with PCS method. The resulting suspension was further diluted with glycerol to prepare an aqueous nanosuspension with amiodarone concentration of 50 mg/mL. The average size of amiodarone particles did not change after the dilution, as verified with PCS method.

### Example 5:

The aqueous nanosuspension of amiodarone prepared as described in example 2 was diluted with human plasma in the ratio 1:1, 1:2, 1:4, 1:8, 1:16, 1:32, 1:64. 1:128, 1:256, 1:512, 1:1024. A commercial preparation (Cordarone^{®}) was diluted in the same manner. The turbidity of the prepared samples was assessed by means of measuring forward scattered light by NepheloStar apparatus. The turbidity of the samples was measured after 5 hours, when the samples reached a stable state (i.e. the amiodarone was no longer precipitating/dissolving). Results are shown in Figure 1. Higher turbidity (i.e. higher RNU value) indicates greater amiodarone precipitation. The turbidity of plasma without amiodarone preparation was around 1700 RNU, indicating that amiodarone in both formulations was practically dissolved in the dilution ratios 1:256 and higher.

From Figure 1 it can observed that the precipitation of amiodarone occurs from the commercial preparation when mixed with human plasma and that precipitated amiodarone gradually dissolves upon further dilution with plasma.

In contrast, amiodarone aqueous nanosuspension prepared in the example 2 and diluted as described in the example 5 exhibits strong turbidity in the beginning due to solid amiodarone particles that are already present in the formulation. These particles gradually dissolve upon dilution with human plasma. As can be deduced from Figure 1, no additional precipitation of the drug occurs upon application of the aqueous nanosuspension.

Moreover, the precipitation of amiodarone in plasma from the commercial preparation results in particles of unspecified size whereas the particles present in the formulation prepared according to the example 2 have a defined size and only get smaller (i.e. they dissolve).

The dilution with plasma has similar effect on the dissolution of amiodarone from aqueous nanosuspension as on the dissolution of amiodarone precipitated from commercial preparation, although the later dissolves faster. Initial lower turbidity of amiodarone upon dilution with plasma is probably the effect of greater amount of surfactants present in the commercial preparation (10% w/w) compared to the formulation prepared in the example 2 (1 % w/w), which allows for greater solubility of amiodarone in the samples.

### Example 6:

Mice (Naval Medical Research Institute, NMRI) were injected with the aqueous nanosuspension prepared according to example 2 and the commercial formulation (Cordarone^{®}) in different doses. The injection was applied via the tail vein. The local changes at the site of injection are shown in Table 1. As can be observed, fewer and less severe adverse effects occurred in the group treated with aqueous nanosuspension formulation prepared according to example 2 than was the case with the commercial formulation.

**Table 1: Tail trauma in mice (NMRI) after injecting different doses of Cordarone^{®} or amiodarone aqueous nanosuspension prepared according to the example 2.**

| Cordarone^{®} | | | | |
|---|---|---|---|---|
| Dose (mg/kg) | No. of animals | No. of deaths | Severity of tail trauma after 3 days | Severity of tail trauma after 6 days |
| 178 | 6 | 0 | 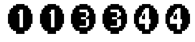 | 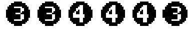 |
| 222.5 | 6 | 1 | 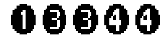 | 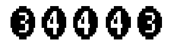 |
| 267 | 6 | 6 | deceased | deceased |
| 356 | 3 | 3 | deceased | deceased |

| Amiodarone aqueous nanosuspension prepared according to example 2 | | | | |
|---|---|---|---|---|
| 151 | 6 | 0 | 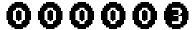 | 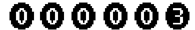 |
| 222.5 | 6 | 0 | 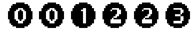 | 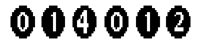 |
| 267 | 6 | 1 | 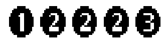 | 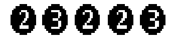 |
| 285 | 5 | 1 | 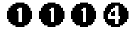 | 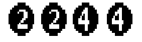 |

| Tail change | Grade |
|---|---|
| No change | 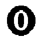 |
| Erythema, mild edema | 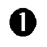 |
| Weal, with/without edema | 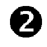 |
| Necrosis along the injection vein | 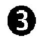 |
| Tail necrosis, tail loss | 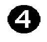 |

### Example 7:

In a glass beaker 2.5 g of polysorbate 80 are mixed with 35 g of water for injections to prepare a clear, yellowish solution. 12.5 g of diazepam are suspended in the prepared polysorbate solution and milled in a high pressure homogenizer at 300 bar for 2 cycles, 1000 bar for 2 cycles and 1500 bar for 20 cycles. The prepared aqueous nanosuspension has an average particle size of 500 nanometers as measured with a photon correlation spectroscopy (PCS) method.

### Example 8:

In a glass beaker 2 g of polysorbate 80 are mixed with 10 g of water for injections to prepare a clear, yellowish solution. 10 g of diazepam are suspended in the prepared polysorbate solution and milled in a wet ball mill to prepare an aqueous nanosuspension with average particle size of around 400 nanometers measured with PCS method.

### Example 9:

In a glass beaker 2 g of poloxamer 188 are mixed with 10 g of water for injections to prepare a clear solution. 10 g of diazepam are suspended in the prepared poloxamer solution and milled in a wet ball mill to prepare an aqueous nanosuspension with average particle size of around 300 nanometers measured with PCS method.

### Example 10:

In a glass beaker 2 g of polysorbate 80 are mixed with 10 g of water for injections to prepare a clear, yellowish solution. 10 g of clarithromycin are suspended in the prepared polysorbate solution and milled in a wet ball mill to prepare an aqueous nanosuspension with average particle size of around 350 nanometers measured with PCS method.

### Example 11:

In a glass beaker 1 g of PVA (polyvinyl alcohol) is mixed with 10 g of water for injections to prepare a clear, yellowish solution. 10 g of clarithromycin are suspended in the prepared polyvinyl alcohol solution and milled in a wet ball mill to prepare an aqueous nanosuspension with average particle size of around 350 nanometers measured with PCS method.

### Methods of analysis

### Photon correlation spectroscopy (PCS) method

Particle size of aqueous nanosuspension was measured by dynamic light scattering technique (also known as photon correlation spectroscopy) using Zetasizer Nano ZS ZEN 3600 (4mW He-Ne laser, 633nm) from Malvern Instruments, UK. Using this technique particle size is obtained by measuring Brownian motion of particles in the sample and then interpreting a size from this using established theories. It does this by illuminating the particles with a laser and analysing the intensity fluctuation in the scattered light. Scattering light was detected at 173° at automatically adjusted attenuator, and temperature 25 °C. Using Zetasizer Nano software, quality reports, which are displayed as a warning message ("result meets/does not meet quality criteria"), were also checked after each measurement. This report reflexes the suitability of the sample for DLS measurement, and reliability of the data obtained, since it is based on the collection of measurement parameters that need to be examined to obtain an adequate degree of confidence regarding the measurement result. The instrument was routinely checked and calibrated using standard reference latex dispersion (Malvern Zeta potential transfer standards, Malvern, UK).

### Determination of turbidity - Scattered light

Samples were added in different dilution ratios to microtiter wells, where they were shaked for 10s to assure uniformity of the samples. The turbidity measurement was performed immediatelly after shaking in 96-microtiter plates by using Nephelostar Galaxy (BMG Labtech) with the following set up: laser intensity 90%, beam focus 1.5mm, measured time per well 0.1 second. Results are shown as mean values of 2-3 independent measurements.

## Claims

1. An aqueous nanosuspension for use in intravenous administration comprising an active ingredient selected from a group consisting of amiodarone, diazepam and clarithromycin, and a stabilizing agent, wherein the stabilizing agent is present in an amount of less than 5 % w/w, in a therapeutic use of said selected active ingredient.

2. The aqueous nanosuspension according to Claim 1, wherein the stabilizing agent is present in an amount of less than 2 % w/w.

3. The aqueous nanosuspension according to Claim 1 or 2, wherein the active ingredient is amiodarone.

4. The aqueous nanosuspension according to any of the preceding Claims, wherein the stabilizing agent is selected from the group consisting of polysorbate 80, polyvinyl alcohol and poloxamer 188, preferably polysorbate 80.

5. The aqueous nanosuspension according to any of the preceding Claims, further comprising one or more pharmaceutically acceptable excipients.

6. The aqueous nanosuspension according to Claim 5, wherein the pharmaceutically acceptable excipients are selected from the group consisting of protective agents and viscosity increasing agents.

7. The aqueous nanosuspension according to Claim 6 wherein the protective agent is benzyl alcohol, and the viscosity increasing agent is glycerol.

8. The aqueous nanosuspension according to any of the preceding Claims, wherein the particles have an average size in the range of from 10 nm to 1000 nm, preferably in the range of from 10 nm to 500 nm.

9. The aqueous nanosuspension according to any of the preceding Claims, wherein said aqueous nanosuspension is stable in a pH range from 2 to 10, preferably in a pH range from 5 to 8.

10. A process for preparing an aqueous nanosuspension according to Claim 1, the process comprising:
(i) providing an active ingredient selected from a group consisting of amiodarone, diazepam and clarithromycin,
(ii) providing aqueous solution of stabilizing agent and optionally one or more pharmaceutically acceptable excipients,
(iii) suspending said active ingredient selected from a group consisting of amiodarone, diazepam and clarithromycin in the obtained aqueous solution of stabilizing agent and optionally one or more pharmaceutically acceptable excipients, and
(iv) subjecting the suspension obtained at step (iii) to high pressure and/or physical force to provide said aqueous nanosuspension.

11. The process according to Claim 10, wherein the active ingredient is amiodarone.

12. The process according to Claims 10 or 11, wherein the stabilizing agent is selected from the group consisting of polysorbate 80, polyvinyl alcohol and poloxamer 188, preferably polysorbate 80.

13. The process according to Claims 11 and 12, wherein pharmaceutically acceptable excipients are selected from the group consisting of protective agents and viscosity increasing agents, preferably the protective agent is benzyl alcohol, and the viscosity increasing agent is glycerol.

14. The process according to Claims 12 and 13, wherein the particles of the provided aqueous nanosuspension have an average size of from 10 nm to 1000 nm, preferably of from 10 nm to 500 nm.

15. A pharmaceutical composition comprising the aqueous nanosuspension according to any of Claims 1 to 9.
